Europäisches Patentamt

European Patent Office

Office européen des brevets

(19)

(11) EP 0 841 985 B1

(12) **FASCICULE DE BREVET EUROPEEN**

(45) Date de publication et mention
de la délivrance du brevet:
**19.01.2000 Bulletin 2000/03**

(51) Int Cl.$^7$: **B01J 31/02**, B01J 29/18,
C07C 209/16

(21) Numéro de dépôt: **96923787.4**

(22) Date de dépôt: **05.07.1996**

(86) Numéro de dépôt international:
**PCT/BE96/00072**

(87) Numéro de publication internationale:
**WO 97/02891 (30.01.1997 Gazette 1997/06)**

(54) **CATALYSEUR, SON PROCEDE DE FABRICATION ET SON UTILISATION DANS UN PROCEDE DE FABRICATION DE METHYLAMINES**

KATALYST, HERSTELLUNGSVERFAHREN UND VERWENDUNG IN EINEM VERFAHREN ZUR HERSTELLUNG VON METYHLAMINEN

CATALYST, PREPARATION PROCESS THEREOF AND ITS USE IN A METHOD FOR MAKING METHYLAMINES

(84) Etats contractants désignés:
**AT BE CH DE DK ES FI FR GB GR IE IT LI LU MC NL PT SE**
Etats d'extension désignés:
**AL LT LV SI**

(30) Priorité: **07.07.1995 GB 9513943**

(43) Date de publication de la demande:
**20.05.1998 Bulletin 1998/21**

(73) Titulaire: **UCB, S.A.**
**1070 Bruxelles (BE)**

(72) Inventeurs:
• **VAN GYSEL, August**
**F-59740 Solre le Château (FR)**
• **PASSELECQ, Jean**
**B-1420 Braine-l'Alleud (BE)**

(56) Documents cités:
**EP-A- 0 036 109**      **EP-A- 0 199 854**
**EP-A- 0 593 086**      **EP-A- 0 600 483**
**US-A- 3 980 586**      **US-A- 4 582 650**

Printed by Jouve, 75001 PARIS (FR)

**Description**

La présente invention se rapporte à un procédé de préparation d'une mordénite sous forme ammonium modifiée, et en particulier à un catalyseur comprenant ladite mordénite sous forme ammonium modifiée préparée par ce procédé, de même qu'à un procédé de fabrication de méthylamines par réaction en phase gazeuse du méthanol avec l'ammoniac à température élevée et éventuellement sous pression élevée, dans lequel on utilise ledit catalyseur.

Dans la synthèse catalytique des méthylamines au départ d'ammoniac et de méthanol, on prépare d'abord un mélange vaporisé de méthanol et d'ammoniac que l'on fait ensuite réagir dans un réacteur à des températures d'environ 220 à environ 500°C, sous des pressions comprises entre la pression atmosphérique et environ 50 bars, par passage sur un lit de catalyseur.

Le produit de la réaction entre l'ammoniac et le méthanol est constitué par un mélange des trois amines, la monométhylamine (en abrégé MMA), la diméthylamine (en abrégé DMA), la triméthylamine (en abrégé TMA), de l'eau, de l'ammoniac et du méthanol n'ayant pas réagi. En outre, du diméthyléther (en abrégé DME) peut se former comme produit secondaire. Parmi ces produits, la diméthylamine est l'amine la plus recherchée sur le plan industriel; elle sert en effet de matière première pour la fabrication de nombreux produits commerciaux, tels que des solvants, des produits pharmaceutiques, des accélérateurs de vulcanisation, des surfactants, des fongicides (par exemple le disulfure de tétraméthylthiurame), etc. La production de diméthylamine à partir d'ammoniac et de méthanol implique nécessairement une étape de séparation des produits obtenus après la réaction. Cependant l'isolation de la diméthylamine par distillation à partir du mélange de méthylamines est considérablement compliquée par le fait que l'ammoniac résiduel et les méthylamines produites forment des mélanges azéotropiques.

A l'heure actuelle, les méthylamines sont fabriquées à l'échelle industrielle à partir de méthanol et d'ammoniac en utilisant le plus souvent des catalyseurs de silice-alumine amorphe, parce que ces catalyseurs sont doués d'excellentes propriétés catalytiques.

Toutefois, le mélange de méthylamines obtenu en présence de ces catalyseurs contient une proportion majeure de triméthylamine et dès lors la production de la diméthylamine souhaitée est insuffisante.

C'est pourquoi de nombreuses recherches ont été faites afin de trouver des catalyseurs qui permettent d'obtenir sélectivement la diméthylamine, en supprimant le plus possible la production de triméthylamine. Les quantités des différentes méthylamines dans le produit de réaction sont déterminées par l'équilibre thermodynamique de la réaction; ces quantités dépendent entre autres de la température de réaction et du rapport molaire des réactifs. Par exemple, pour une température de réaction de 300°C, une alimentation en ammoniac et méthanol correspondant à un rapport atomique N/C de 2:1 et un débit d'alimentation du méthanol de 0,3 kg/h/kg de catalyseur, la composition en % en poids du mélange de méthylamines à l'équilibre pour un catalyseur actif mais non sélectif est de 20 % de MMA, 22,5 % de DMA et 57,5 % de TMA pour une conversion totale du méthanol. Cette composition de produits correspond à la composition à l'équilibre thermodynamique. La triméthylamine est donc produite en quantité majeure dans ces conditions.

En revanche, si la production de triméthylamine était supprimée, la composition en % en poids du mélange de MMA et de DMA à l'équilibre serait de 31,5% de MMA et de 68,5% de DMA dans les mêmes conditions à conversion totale du méthanol.

On voit immédiatement l'intérêt de mettre au point des catalyseurs sélectifs qui permettent de supprimer quasi totalement la production de triméthylamine.

On a proposé un grand nombre de catalyseurs en vue d'atteindre cet objectif. La littérature mentionne en particulier les zéolites synthétiques ou naturelles, comme par exemple les zeolites X, Y, ZK-5, ZSM-5, ZSM-12, FU-1, SK, l'érionite, la ferriérite, la faujasite, la chabasite, la clinoptilolite et plus particulièrement la mordénite. Ces zéolites ont été utilisées soit telles quelles, soit après avoir subi divers traitements pour en modifier les caractéristiques, comme le nombre de sites acides, la taille des pores ou le rapport silicium/aluminium. Parmi les traitements proposés, on trouve entre autres la modification de la nature et de la proportion des cations, le traitement par des acides, la calcination avec ou sans la présence de vapeur d'eau ou encore la silylation au moyen de divers agents de silylation. Les divers traitements proposés peuvent entraîner une certaine amélioration de l'activité catalytique et/ou de la sélectivité pour la production de la monométhylamine ou de la diméthylamine.

Toutefois, en utilisant ces zéolites comme catalyseurs, la sélectivité pour la production de la diméthylamine reste en général relativement faible. Très souvent, les valeurs observées sont très proches des valeurs que l'on peut atteindre lorsque l'équilibre thermodynamique est réalisé entre les trois méthylamines.

Ainsi, dans un article relatif à la synthèse sélective de diméthylamine à partir de méthanol et d'ammoniac en présence de diverses zéolites (I. MOCHIDA et al., J. Catal., 82, (1983), 313-321 ), les auteurs rapportent que les meilleurs résultats de conversion du méthanol et de sélectivité en diméthylamine sont obtenus avec des mordénites (Zeolon de la société NORTON Co. Ltd), sous forme protonique ou sous une forme dans laquelle les cations sont échangés par des cations sodium, magnésium ou lanthane-hydrogène. Sous cette dernière forme, on obtient, à une température de réaction de 400 °C, un taux de conversion du méthanol qui s'élève à 94,5 % en moles, et une sélectivité en DMA de 51,5 % en moles. La quantité formée de triméthylamine est toutefois loin d'être négligeable, vu que la sélectivité en TMA s'élève à 11,9 % en moles.

Dans les brevets américains US-A-5.137.854 et US-A-5.210.308, on propose de remédier à cet inconvénient par l'utilisation d'une mordénite sous forme protonique qui a subi un traitement particulier. Ce traitement consiste à soumettre la mordénite sous forme sodique à un traitement avec du tétrachlorosilane, en phase gazeuse, à température élevée (environ 700 °C dans l'exemple) et à convertir ensuite la mordénite sous forme sodique ainsi traitée en une mordénite sous forme protonique par échange d'ions. Pour cet échange, les ions sodium de la mordénite traitée sont échangés par des ions ammonium et la mordénite sous forme ammonium ainsi obtenue est soumise à une calcination pendant plusieurs heures à 450 °C. D'après ces brevets, ce traitement de la mordénite ne modifie pas sensiblement son rapport Si/Al, autrement dit, la mordénite ne subit pas de désalumination. Cette mordénite modifiée permet d'obtenir un taux de conversion du méthanol de 98,9 % en moles et une sélectivité en diméthylamine de 61,2 % en moles. Le procédé décrit dans ces brevets procure donc un bon taux de conversion du méthanol et une bonne sélectivité en diméthylamine. Toutefois, la production de triméthylamine reste importante (voir l'exemple 4 ci-après).

Dans la demande de brevet européen EP-A-0.593.086, on a tenté également de résoudre ce problème. On y propose un procédé de préparation de méthylamines qui permet de produire sélectivement la monométhylamine et la diméthylamine et de réduire la production de triméthylamine à quelques %. A cet effet, la synthèse des méthylamines est effectuée en présence d'un catalyseur spécifique. Ce catalyseur est formé en soumettant une mordénite sous forme protonique d'abord à un ou plusieurs traitements de silylation avec un agent de silylation en phase liquide et ensuite à un traitement thermique à une température de 300 à 600 °C en présence d'air ou d'oxygène. Le traitement de silylation est effectué par dispersion de la mordénite dans une solution de l'agent de silylation dans un solvant. Cependant, avant le traitement de silylation, la teneur en eau de la mordénite doit être réglée à une valeur prédéterminée. Ainsi, lorsque le solvant utilisé est soluble dans l'eau (par exemple un alcool), la mordénite est calcinée à une température de 350 à 600 °C jusqu'à ce que sa teneur en eau soit de 4 % en poids ou moins. Lorsque le solvant utilisé n'est pas miscible avec l'eau (benzène, etc.), la mordénite doit contenir de 3 à 40 % en poids d'eau avant d'être traitée par l'agent de silylation; cette teneur en eau est obtenue soit par séchage contrôlé de la mordénite, soit par calcination et conditionnement dans une atmosphère humide.

Ce procédé permet d'atteindre une très bonne sélectivité en diméthylamine (environ 64%). Toutefois, le taux de conversion du méthanol n'est que d'environ 90%, ce qui signifie qu'à l'échelle industrielle, l'application de ce procédé nécessitera le recyclage d'une quantité non négligeable de méthanol non converti. En outre, ces résultats sont obtenus par une technologie élaborée et délicate. En effet, dans la préparation du catalyseur interviennent toujours plusieurs calcinations à température élevée en présence d'air; de plus, avant le traitement de silylation, la teneur en eau de la mordénite doit être scrupuleusement contrôlée, sans quoi il n'est pas possible de maintenir la production de la triméthylamine à un faible niveau (voir en particulier les exemples 3, 6 et 8 de cette demande de brevet).

Par conséquent, malgré la disponibilité de nombreux catalyseurs pour la synthèse catalytique des méthylamines à partir d'ammoniac et de méthanol, il subsiste un besoin de trouver un catalyseur qui soit à la fois

(a) très sélectif pour la production de la monométhylamine et surtout de la diméthylamine souhaitée (permettant d'atteindre par exemple des sélectivités en diméthylamine de 70 % en moles et plus), tout en ne formant pratiquement pas de triméthylamine ou de produits secondaires, notamment du diméthyléther;
(b) très actif pour pouvoir conduire la réaction à des taux de conversion du méthanol très élevées, de préférence proches de 100 mole % (pour éviter le recyclage du méthanol);
(c) susceptible d'être préparé par un procédé aisément et économiquement réalisable à l'échelle industrielle.

On a découvert maintenant de façon tout à fait surprenante qu'il est possible de préparer un catalyseur très actif et extrêmement sélectif au départ d'une mordénite sous forme ammonium, lorsqu'on fait subir à cette mordénite un traitement particulier de silylation avec du tétrachlorosilane en phase gazeuse dans des conditions déterminées.

On a trouvé en effet qu'en utilisant ce catalyseur spécifique à base de mordénite sous forme ammonium, dans la synthèse des méthylamines, on obtient d'excellentes sélectivités en diméthylamine dépassant 70 % en moles et qui peuvent même atteindre 81,6 % en moles, quasi sans formation de triméthylamine et de diméthyléther, et avec des taux de conversion du méthanol proches de 100 % en moles.

Dès lors, un objet de la présente invention est un procédé de préparation d'une mordénite sous forme ammonium modifiée, caractérisé en ce qu'il comprend

(1) le séchage d'une mordénite sous forme ammonium à une température inférieure à 400°C, et
(2) le traitement de la mordénite sous forme ammonium séchée ainsi obtenue par du tétrachlorosilane en phase gazeuse, à une température comprise entre 300 et 600°C.

Un autre objet de la présente invention est un catalyseur, comprenant une mordénite sous forme ammonium modifiée obtenue par le procédé précité.

Enfin, encore un autre objet de la présente invention est un procédé de fabrication de méthylamines, caractérisé en ce que l'on fait passer en phase gazeuse, à une température comprise entre 220°C et 500°C, un mélange de méthanol et d'ammoniac sur un catalyseur comprenant une mordénite sous forme ammonium modifiée préparée par le procédé précité.

Le catalyseur utilisé conformément à l'invention dans la synthèse catalytique des méthylamines à partir de méthanol et d'ammoniac est préparé à partir d'une mordénite sous forme ammonium.

Une mordénite est un aluminosilicate cristallin qui, soit existe à l'état naturel, soit est préparé par synthèse chimique. La composition globale des mordénites naturelles peut s'exprimer de manière générale par la formule $Na_8$ $[(AlO_2)_8(SiO_2)_{40}].24H_2O$. Les mordénites naturelles possèdent donc un rapport atomique Si/Al de 5:1. Cependant, on connaît également des mordénites synthétiques dans lesquelles le rapport Si/Al est supérieur à 5:1 (voir pages 321-332 du livre de P. A. Jacobs et J. A. Martens, "Synthesis of High-Silica Aluminosilicate Zeolites", volume 33 de "Studies in Surface Science and Catalysis", Elsevier, 1987), ou dans lesquelles les ions sodium ont été remplacés par des ions hydrogène, alcalino-terreux ou ammonium, ou encore par d'autres ions alcalins.

La mordénite utilisée comme matière de départ pour la préparation du catalyseur selon la présente invention est une mordénite sous forme ammonium, dans laquelle le rapport atomique Si/Al est compris entre 5:1 et 20:1, de préférence entre 5:1 et 12:1. Sa teneur en sodium doit être très faible et de préférence inférieure à 0,1 % en poids par rapport au poids de la mordénite. Les mordénites sous forme ammonium sont disponibles dans le commerce ou peuvent être préparées à partir d'une mordénite renfermant comme cations des ions de métaux alcalins ou alcalino-terreux par échange de ces ions par des ions ammonium. Cet échange d'ions peut être effectué par des méthodes connues en soi, par exemple, par traitement au moyen d'une solution aqueuse de nitrate d'ammonium.

La mordénite sous forme ammonium modifiée qui convient comme catalyseur selon la présente invention est préparée par un procédé qui comporte essentiellement une étape de séchage. suivie d'une étape de silylation.

En ce qui concerne l'étape de séchage, elle doit être menée dans des conditions telles que la mordénice reste sous forme ammonium. On a constaté en effet, qu'au-dessus d'une température de 400°C, la mordénite sous forme ammonium se transforme facilement en mordénite sous forme protonique. C'est pourquoi, l'étape de séchage du procédé selon la présente invention est exécutée généralement à une température inférieure à 400°C, et de préférence à une température dans l'intervalle de 230 °C à 350 °C, dans un courant de gaz inerte sec (azote). La température et la durée du séchage doivent être suffisantes pour éliminer toute l'eau adsorbée sur la mordénite, mais cette température ne doit toutefois pas être trop élevée et la durée du séchage trop longue afin d'éviter de volatiliser l'ammoniac lié chimiquement (chimisorbé). La durée du séchage est généralement de 180 à 540 minutes, de préférence de 360 à 480 minutes.

Selon la présente invention, tout procédé de séchage de la mordénite peut être utilisé. Ainsi, on peut, par exemple, sécher la mordénite sous vide, même à la température ambiante. Le séchage peut donc être exécuté sous une pression inférieure ou supérieure à la pression atmosphérique. De préférence, le séchage est effectué sous une pression pouvant aller jusqu'à 3 bars.

Pendant cette étape de séchage, la quantité d'eau dégagée peut être contrôlée, par exemple, par condensation de celle-ci à la sortie du réacteur, ou par analyse par spectrométrie de masse. On arrête le séchage lorsque le dégagement d'eau est terminé. D'autre part, une analyse des gaz dégagés, par barbotage dans l'eau et détermination par titrage de l'ammoniac présent dans les solutions aqueuses obtenues, a montré que la quantité d'ammoniac libérée pendant le procédé de séchage est inférieure à 0,01 % par rapport au poids de la mordénite mise en oeuvre. La quantité d'ammoniac libérée pendant le séchage est donc négligeable par rapport à la quantité d'ammoniac présent dans la mordénite de départ.

A la fin de cette étape de séchage, on laisse refroidir le produit séché sous un courant de gaz inerte (azote) pour qu'il soit ramené à la température ambiante.

La silylation de la mordénite sous forme ammonium séchée est effectuée au moyen de tétrachlorosilane en phase gazeuse, par chauffage à partir de la température ambiante, en augmentant progressivement la température à la vitesse de 1 à 5°C par minute, de préférence de 2,5 à 4°C par minute, jusqu'à une température de 300 à 600°C, de préférence de 450 à 550°C, et maintien ensuite de cette température pendant une durée de 60 à 180 minutes, de préférence de 120 à 160 minutes. De manière avantageuse, on utilise un mélange gazeux renfermant un gaz inerte et du tétrachlorosilane dans lequel la pression partielle de tétrachlorosilane se situe entre 0.05 et 1,0 bar, en particulier entre 0,2 et 0.6 bar. Lors de cette étape de silylation, une légère désalumination de ia mordénite a lieu: le rapport atomique Si/Al passe d'une valeur initiale comprise entre 5:1 et 20:1 à une valeur comprise entre 10:1 et 30:1 .

Conformément à la présente invention, il est essentiel d'utiliser du tétrachlorosilane comme agent de silylation. On a constaté en effet que la silylation au moyen d'autres agents de silylation (dichlorodiméthylsilane ou polydiméthylsiloxane) ne permet pas d'obtenir un catalyseur sélectif pour la formation de la diméthylamine dans la synthèse catalytique des méthylamines à partir d'ammoniac et de méthanol.

Après refroidissement sous un flux de gaz inerte jusqu'à la température ambiante, la mordénite ainsi traitée est abondamment lavée à l'eau distillée de manière à éliminer les chlorures et sels d'aluminium résiduels. Ce lavage est répété plusieurs fois jusqu'à ce que le pH de la solution surnageante soit neutre. Ensuite, on sèche la mordénite sous forme ammonium modifiée ainsi obtenue à 60 °C jusqu'à poids constant.

Les mordénites sous forme ammonium modifiées utilisées comme catalyseurs selon la présente invention possèdent en général un rapport atomique Si/Al de 10:1 à 30:1, de préférence de 15:1 à 25:1.

Le procédé de préparation de la mordénite sous forme ammonium modifiée selon l'invention est plus simple et moins onéreux que les procédés de l'état de la technique. En effet, contrairement au procédé de préparation de la mordénite modifiée décrit dans les brevets américains US-A-5.137.854 et US-A-5.210.308 qui comporte quatre étapes essentielles, dont deux étapes de calcination, le procédé de préparation de la mordénite sous forme ammonium modifiée conforme à la présente invention se fait en deux étapes (séchage et silylation), sans faire intervenir une étape de calcination. De plus, le procédé selon la présente invention est plus facile à mettre en oeuvre que le procédé selon la demande de brevet européen EP-A-0.593.086, dans lequel il faut veiller scrupuleusement à maintenir la teneur en eau dans la mordénite à une valeur prédéterminée avant l'étape de silylation, et dans lequel on effectue également plusieurs calcinations.

Il s'ensuit que le procédé de préparation de la mordénite sous forme ammonium modifiée selon la présence invention est facilement et économiquement applicable à l'échelle industrielle contrairement aux procédés de l'état de la technique.

Dans le procédé de synthèse des méthylamines selon la présente invention, le méthanol et l'ammoniac utilisés comme matières de départ peuvent être purs, ou pour des raisons économiques évidentes, de qualité technique.

Les matières premières sont utilisées dans le procédé conforme à l'invention en des quantités telles que le rapport atomique azote/carbone (N/C) est de 0,5:1 à 5:1, de préférence de 0,8:1 à 2:1.

Lorsque le rapport atomique N/C augmente, la sélectivité pour la production de diméthylamine a tendance à diminuer, tandis que lorsque le rapport atomique N/C diminue, la production de triméthylamine augmente. C'est pourquoi, il n'est pas recommandé de travailler avec un rapport atomique N/C en dehors de l'intervalle de 0,5:1 à 5:1.

Le débit d'alimentation du méthanol est avantageusement compris entre 0,1 et 2 kg/h/kg de catalyseur.

Les conditions opératoires appliquées dans le procédé de l'invention sont celles généralement utilisées dans la fabrication des méthylamines par réaction catalytique d'ammoniac avec du méthanol en phase gazeuse. On opère généralement dans un intervalle de température compris entre 220 et 350°C, de préférence entre 280 et 320°C, sous une pression allant de la pression atmosphérique jusqu'à environ 100 bars, de préférence allant d'environ 1 à 50 bars.

On n'impose pas de restrictions quant à la nature de l'appareillage utilisé pour exécuter le procédé de l'invention. Le procédé peut être conduit de manière continue ou discontinue. Le lit catalytique peut être un lit fixe ou fluidisé.

A la sortie du réacteur, le mélange gazeux est séparé en ses divers constituants par des méthodes connues en soi, par exemple par distillation fractionnée. Après séparation des divers constituants de l'effluent du réacteur, on peut, si on le désire, recycler partiellement ou totalement l'ammoniac, la monométhylamine et/ou la diméthylamine.

Dans le procédé conforme à l'invention, dans lequel on utilise comme catalyseur la mordénite sous forme ammonium modifiée préparée comme décrit précédemment, on obtient très facilement des sélectivités très élevées pour la production de diméthylamine, qui peuvent atteindre de 72 à 82 % en moles, avec des taux de conversion du méthanol proches de 100 % en moles. En outre, il ne se forme pratiquement pas de triméthylamine, ni des produits secondaires comme le diméthyléther.

De plus, on obtient les mêmes bons résultats lorsque le mélange gazeux de méthanol et d'ammoniac contient de la monométhylamine et/ou de la diméthylamine et/ou de la triméthylamine. Les méthylamines formées peuvent donc être recyclées avec l'ammoniac dans le mélange gazeux alimenté au réacteur sans affecter la production sélective de la diméthylamine, ce qui constitue un atout industriel important. Enfin, comme il est démontré plus loin dans les exemples, le catalyseur conserve son activité et sa sélectivité pendant une longue durée.

Les exemples qui suivent vont illustrer la présente invention sans la limiter. Dans ces exemples, les rapports atomiques Si/Al cités ont été déterminés par résonance magnétique nucléaire du $^{29}$A1 par rotation à l'angle magique ("Magic Angle Spinning Nuclear Magnetic Resonance" aussi appelé MAS NMR), en utilisant comme référence une mordénite contenant uniquement des atomes d'aluminium occupant des sites tétraédriques, et dont la teneur en aluminium a été déterminée par la technique du plasma inductif couplé, comme décrit par D.R. Corbin et al. dans Anal. Chem. 59 (1987), 2722-2728.

Exemple 1. Préparation du catalyseur conforme à l'invention.

Dans cet exemple, on met en oeuvre la mordénite PQ Zeolite CBV 20A (The PQ Corporation, Valley Forge, Etats-Unis) qui est une mordénite synthétique sous forme ammonium, à faible teneur en Na (0,01% en poids par rapport au poids de la mordénite). Cette mordénite possède un rapport atomique Si/Al de 10:1, et une teneur en azote sous forme d'ammoniac de 1.95 % en poias. La teneur en ammoniac a été déterminée par déplacement des ions $NH_4^+$

de la mordénite par une solution aqueuse concentrée d'hydroxyde de sodium, suivi de l'entraînement à la vapeur de l'ammoniac formé, de la condensation de la vapeur, et du dosage de l'ammoniac dans la solution aqueuse ainsi obtenue.

(1) Séchage.

Environ 25 g de mordénite sont pastillés, puis passés à travers un tamis, pour donner 10 g de pastilles ayant une granulométrie comprise entre 250 et 500 μm. La fraction de mordénite de cette dimension de particule est introduite dans un tube en quartz de 25 mm de diamètre muni d'une gaine thermométrique. La partie de tube utilisée est chauffée extérieurement par un enroulement électrique disposé de manière à assurer une température uniforme dans le lit de catalyseur. On chauffe la mordénite à la pression atmosphérique jusqu'à 300°C à la vitesse de 2,5°C/min, dans un courant d'azote à un débit de 40 ml/min et on maintient la mordénite à cette température de 300°C pendant 5 heures. Ensuite on laisse refroidir sous un courant d'azote sec jusqu'à la température ambiante.

L'analyse montre que la quantité d'ammoniac libérée pendant cette étape de séchage est négligeable par rapport au poids de la mordénite mise en oeuvre.

(2) Traitement au tétrachlorosilane.

On fait passer un courant d'azote à travers un barboteur contenant du tétrachlorosilane, maintenu à une température assurant une pression partielle de tétrachlorosilane d'environ 0,24 bar. Puis, on amène le mélange gazeux d'azote et de tétrachlorosilane sur le lit de catalyseur (mordénite séchée) à un débit de 40 ml/min. le lit de catalyseur étant progressivement chauffé jusqu'à 550°C à la vitesse de 3,5°C/min. On maintient la mordénite encore pendant 2 heures à 550°C sous le courant gazeux, puis on laisse refroidir sous azote jusqu'à la température ambiante.

On met la mordénite ainsi traitée en suspension dans 2 litres d'eau distillée, on décante l'eau et on répète cette opération jusqu'à ce que le pH de la solution surnageante soit neutre. Ensuite, on sèche la mordénite ainsi obtenue à 60°C jusqu'à poids constant. La mordénite sous forme ammonium modifiée ainsi obtenue possède un rapport atomique Si/Al de 25:1. Sa teneur en azote sous forme d'ammoniac est de 0,7 % en poids. La teneur en ammoniac est déterminée par déplacement des ions $NH_4^+$ par une solution aqueuse concentrée d'hydroxyde de sodium, suivi de l'entraînement à la vapeur de l'ammoniac formé, de la condensation de la vapeur, et du dosage de l'ammoniac dans la solution aqueuse ainsi obtenue.

Cette teneur en azote pour une mordénite ayant un rapport atomique Si/Al de 25:1 signifie que la mordénite obtenue après silylation est toujours sous forme ammonium. En effet, la formule générale théorique d'une telle mordénite devrait être $(NH_4)_{1,85}[(AlO_2)_{1,85}(SiO_2)_{46,15}] \cdot 24H_2O$, et son poids moléculaire théorique serait donc de 3550. La teneur en azote théorique exprimée en pour-cent en poids par rapport au poids total de la mordénite est donc de $[(14 \times 1,85)/3550] \times 100 = 0,73\%$ en poids. Comme la teneur expérimentale en azote est de 0,7 % en poids par rapport au poids de la mordénite, il est clair que la mordénite est toujours sous forme ammonium après le traitement de silylation selon l'invention.

Exemple 2 (comparatif). Préparation de catalyseurs à base de diverses zéolites.

Dans cet exemple, le procédé mis en oeuvre est le même qu'à l'exemple 1, mais il est appliqué à diverses zéolites commerciales ou synthétiques. La préparation des catalyseurs à base de ces diverses zéolites a pour but de comparer leurs performances dans la synthèse des méthylamines à partir d'ammoniac et de méthanol, avec les performances de la mordénite sous forme ammonium modifiée préparée selon le procédé de l'invention (voir exemple 4 ci-après).

2.1. Silylation de la zéolite Beta $H^+$.

On opère exactement comme dans la préparation du catalyseur de l'exemple 1, mais on remplace la mordénite de départ par la zéolite Beta $H^+$ CP 811-25 (PQ Zeolites B.V., Leiden, Pays-Bas), qui est une zéolite synthétique sous forme protonique dont le rapport atomique Si/Al est de 13:1. La zéolite Beta sous forme protonique modifiée ainsi obtenue possède un rapport Si/Al de 150:1.

2.2. Silylation de la zéolite Beta $Na^+$.

a) La zéolite Beta $H^+$ CP 811-25 utilisée à l'exemple 2.1 ci-dessus est transformée en zéolite Beta sous forme sodique par agitation dans une solution aqueuse de $NH_3$ à la température ambiante, suivie d'une agitation dans une solution aqueuse de chlorure de sodium à la température de reflux pendant 4 heures. Elle est ensuite lavée à l'eau distillée jusqu'à disparition des ions chlorures. puis séchée à 60 °C.

b) Ensuite, la zéolite Beta sous forme sodique préparée en 2.2.a) est séchée et traitée exactement comme à l'exemple 1, pour donner une zéolite Beta $Na^+$ modifiée dont le rapport atomique Si/Al est de 40:1.

2.3. Silylation d'une mordénite sous forme sodique.

a) La mordénite PQ Zéolite CBV 20A sous forme ammonium utilisée à l'exemple 1 est transformée en la forme sodique par agitation dans une solution aqueuse de chlorure de sodium à la température de reflux pendant 4 heures. Elle est ensuite lavée à l'eau distillée jusqu'a disparition des ions chlorures, puis séchée à 60 °C. On obtient une mordénite sous forme sodique dont le rapport atomique Si/Al est de 10:1.

b) Ensuite, la mordénite sous forme sodique préparee en 2.3.a) est séchée et traitée exactement comme décrit à l'exemple 1, pour donner une mordénite sous forme sodique modifiée dont le rapport atomique Si/Al est de 17:1.

Exemple 3 (comparatif). Préparation du catalyseur décrit dans le brevet américain US-A-5.137.854.

A titre comparatif, on a également préparé une mordénite sous forme protonique modifiée selon le procédé décrit dans le brevet américain US-A-5.137.854.

3.1. La mordénite PQ Zéolite CBV 20A sous forme ammonium utilisée à l'exemple 1 est transformée en la forme sodique par agitation dans une solution aqueuse de chlorure de sodium à la température de reflux pendant 4 heures. Elle est ensuite lavée à l'eau distillée jusqu'à disparition des ions chlorures, puis séchée à 60 °C. On obtient une mordénite sous forme sodique qui possède un rapport atomique Si/Al de 10:1.

3.2. Ensuite, on a reproduit exactement le mode de préparation du catalyseur décrit à l'exemple du brevet américain 5.137.854 (colonne 6, ligne 50, jusqu'à la colonne 7, ligne 34), en utilisant la mordénite sous forme sodique préparée en 3.1 ci-dessus. Dans ce procédé, on chauffe la mordénite sous forme sodique dans un courant d'azote à 700°C pendant 30 minutes, puis on la soumet à cette température à un courant gazeux de tétrachlorosilane et d'azote pendant environ 3 heures; on lave la mordénite ainsi traitée avec de l'eau distillée jusqu'à disparition des ions chlorures, on la soumet à une calcination à 450°C, on échange les ions sodium de la mordénite par des ions ammonium par traitement au moyen d'une solution de $NH_4NO_3$, et on soumet finalement la mordénite sous forme ammonium ainsi obtenue à une nouvelle calcination pendant 2 heures à 450°C. On obtient une mordénite sous forme protonique modifiée possédant un rapport atomique Si/Al de 10:1.

Exemple 4. Synthèse de méthylamines.

Dans cet exemple, on compare les performances catalytiques d'une mordénite sous forme ammonium modifiée préparée selon le procédé de l'invention (cf. exemple 1) avec celles de diverses autres zéolites traitées ou non par du tétrachlorosilane.

4.1 Appareillage et conditions opératoires.

Le réacteur utilisé est un tube en verre Pyrex, d'une hauteur de 50 cm et d'un diamètre intérieur d'environ 22 mm, traversé au centre par une gaine thermométrique dans laquelle coulisse un thermocouple permettant une surveillance étroite de la température du catalyseur.

Ce tube est disposé dans un bain de sable fluidisé chauffé de l'extérieur par une résistance électrique de manière à assurer une distribution uniforme de la température. Dans le tube, la couche du catalyseur soumis à l'essai est précédée par une couche de matière inerte (α-alumine) qui sert au préchauffage des réactifs gazeux avant leur passage sur le lit catalytique.

Pour les essais, on utilise 7 grammes de catalyseur en pastilles broyées ayant une granulométrie comprise entre 300 et 400 µm; le catalyseur est en outre dilué par une matière inerte.

Le mélange gazeux d'ammoniac et de méthanol alimenté au réacteur parcourt celui-ci de haut en bas. On effectue la réaction à une température de 300 °C, à la pression atmosphérique et avec un débit d'alimentation du méthanol de 0,3 kg/h/kg de catalyseur. Le débit d'alimentation de l'ammoniac est tel que l'on réalise un rapport atomique N/C de 2:1.

L'expérience montre que ces essais effectués à la pression atmosphérique à partir d'un mélange gazeux de méthanol, d'ammoniac et éventuellement de méthylamines recyclées constituent une bonne méthode de comparaison des catalyseurs ainsi qu'une base fiable pour l'extrapolation à des conditions de pressions plus élevées.

A la sortie du réacteur, un débit connu d'azote dilue les produits gazeux sortant et évite la condensation de ceux-ci. L'analyse de la composition du mélange gazeux est effectuée par chromatographie en phase gazeuse.

4.2. Comparaison des performances des catalyseurs.

Les résultats obtenus pour chaque catalyseur testé dans la synthèse des méthylamines dans les conditions décrites à l'exemple 4.1., sont reportés au tableau I ci-après.

Dans ce tableau, les termes utilisés ont la signification suivante:

- **-** zéolite Beta H$^+$: zéolite Beta H$^+$ CP 811-25 vendue par PQ Zeolites B.V., Leiden, Pays-Bas;
- **-** zéolite Beta H$^+$.SiCl$_4$: zéolite silylée préparée à l'exemple 2.1;
- **-** zéolite Beta Na$^+$.SiCl$_4$: zéolite silylée préparée à l'exemple 2.2.;
- **-** zéolite ZSM-12 NH$_4$$^+$ calcinée: une zéolite sous forme sodique, obtenue selon la méthode décrite à la page 13 (exemple 6a) de l'ouvrage de P.A. Jacobs et J.A. Martens "Synthesis of High-Silica Aluminosilicate Zeolites", volume 33 de "Studies in Surface Science and Catalysis", Elsevier, 1987, puis échangée par une solution aqueuse de NH$_3$ à la température ambiante et calcinée en présence d'air pendant 4 heures à 500 °C.
- **-** mordénite NH$_4$$^+$ calcinée: mordénite PQ =eolite CBV 20A (The PQ Corporation, Valley Forge, Etats-Unis), calcinée en présence d'air pendant 4 heures à 500 °C.
- **-** mordénite 100 H$^+$: une mordénite commerciale sous forme protonique fortement désaluminée, qui possède un rapport Si/Al de 100:1 (vendue par ZEOCAT 44550, Montoir de Bretagne, France);
- **-** mordénite Na$^+$.SiCl$_4$: la mordénite silylée préparée à l'exemple 2.3.;
- **-** mordénite SiCl$_4$.H$^+$: la mordénite silylée préparée à l'exemple 3 selon le procédé décrit dans le brevet américain 5.137.854.
- **-** mordénite NH$_4$$^+$.SiCl$_4$: la mordénite sous forme ammonium modifiée, préparée à l'exemple 1 (conforme à l'invention);
- **-** Si/Al: le rapport atomique Si/Al du catalyseur utilisé;
- **-** C$_{MeOH}$: le taux de conversion du méthanol (% en moles) calculé par la formule

$$C_{MeOH} = 100 - \frac{\text{moles de méthanol non transformé}}{\text{moles de méthanol alimenté}} \times 100 ;$$

- **-** S$_{DME}$: la sélectivité en diméthyléther (% en moles), calculée par la formule

$$S_{DME} = \frac{2 \times \text{moles de DME}}{2 \times \text{moles de DME} + 1 \times \text{moles de MMA} + 2 \times \text{moles de DMA} + 3 \times \text{moles de TMA}} \times 100 ;$$

- **-** S$_{MMA}$: la sélectivité en monométhylamine (% en moles), calculée par la formule

$$S_{MMA} = \frac{1 \times \text{moles de MMA}}{2 \times \text{moles de DME} + 1 \times \text{moles de MMA} + 2 \times \text{moles de DMA} + 3 \times \text{moles de TMA}}$$

- **-** S$_{DMA}$: la sélectivité en diméthylamine (% en moles), calculée par la formule

$$S_{DMA} = \frac{2 \times \text{moles de DMA}}{2 \times \text{moles de DME} + 1 \times \text{moles de MMA} + 2 \times \text{moles de DMA} + 3 \times \text{moles de TMA}} \times 100 ;$$

- **-** S$_{TMA}$: la sélectivité en triméthylamine (% en moles), calculée par la formule

$$S_{TMA} = \frac{3 \times \text{moles de TMA}}{2 \times \text{moles de DME} + 1 \times \text{moles de MMA} + 2 \times \text{moles de DMA} + 3 \times \text{moles de TMA}} \times 100 ;$$

Dans ces formules,

- **-** MeOH = méthanol
- **-** DME = diméthyléther
- **-** MMA = monométhylamine
- **-** DMA = diméthylamine
- **-** TMA = triméthylamine

TABLEAU I

| Catalyseur | Si/Al | C$_{MeO}$ (% en moles) | S$_{DME}$ (% en moles) | S$_{MMA}$ (% en moles) | S$_{DMA}$ (% en moles) | S$_{TMA}$ (% en moles) |
|---|---|---|---|---|---|---|
| zéolite Beta H$^+$ | 13:1 | >99 | 0,5 | 32 | 24 | 44 |

TABLEAU I   (suite)

| Catalyseur | Si/Al | C$_{MeO}$ (% en moles) | S$_{DME}$ (% en moles) | S$_{MMA}$ (% en moles) | S$_{DMA}$ (% en moles) | S$_{TMA}$ (% en moles) |
|---|---|---|---|---|---|---|
| zéolite Beta H$^+$.SiCl$_4$ | 150:1 | 81 | 12* | | 12 | 76 |
| zéolite Beta Na$^+$.SiCl$_4$ | 40:1 | 96 | 17* | | 22 | 61 |
| zéolite ZSM-12 NH$_4^+$ calcinée | 45:1 | 86 | 12* | | 23 | 65 |
| mordénite NH$_4^+$ calcinée | 8:1 | >98 | 0,5 | 15 | 25 | 60 |
| mordénite 100 H$^+$ | 100:1 | 93 | 21* | | 21 | 58 |
| mordénite Na$^+$.SiCl$_4$ | 17,2:1 | >98 | 2 | 38 | 38 | 22 |
| mordénite SiCl$_4$.H$^+$ | 10:1 | 97 | 2 | 20 | 51 | 27 |
| mordénite NH$_4^+$.SiCl$_4$ (selon l'invention) | 25:1 | 97 | 0,4 | 27,2 | 72,1 | 0,3 |

* S$_{DME}$ + S$_{MMA}$

Le tableau I montre clairement que les zéolites Beta et ZSM-12 favorisent la production de la triméthylamine au détriment de la production de la diméthylamine. En fait, les compositions en méthylamines obtenues avec ces zéolites sont très proches des valeurs que l'on peut atteindre théoriquement lorsque l'équilibre thermodynamique est réalisé entre les trois méthylamines. Ces zéolites ne sont donc pas très sélectives pour la production de la diméthylamine.

Par ailleurs, on voit que les mordénites présentent en général une très bonne activité avec des taux de conversion élevés du méthanol. Les mordénites sous forme protonique non silylées mordénite NH$_4^+$ calcinée et mordénite 100 H$^+$), favorisent également la formation de la triméthylamine plutôt que de la diméthylamine. La mordénite sous forme sodique silylée (mordénite Na$^+$.SiCl$_4$), est plus sélective pour la production de la diméthylamine (38 % en moles). Cependant, la sélectivité en triméthylamine reste élevée (22 % en moles).

La mordénite sous forme sodique traitée au SiCl$_4$ et transformée en mordénite protonique, préparée selon le procédé décrit dans les brevets américains 5.137.854 et 5.210.308, est très sélective pour la production de diméthylamine (51 % en moles). Toutefois, la production de la triméthylamine reste importante, vu que la sélectivité en triméthylamine est de 27 % en moles.

Le tableau I montre clairement la supériorité du catalyseur à base de mordénite préparé selon le procédé de l'invention par rapport aux catalyseurs à base d'autres zéolites, traitées ou non par du SiCl$_4$. En effet, la mordénite sous forme ammonium traitée selon l'invention avec du tétrachlorosilane en phase gazeuse à température élevée constitue le catalyseur le plus sélectif pour la production de diméthylamine, avec une sélectivité en DMA de 72,1 % en moles; de plus, avec ce catalyseur, la production de triméthylamine et de diméthyléther est extrêmement faible (0,7 % en moles pour l'ensemble de ces deux produits) et la conversion du méthanol est presque complète (97 % en moles).

Exemple 5. Influence du rapport atomique N/C

Dans cet exemple, on fait varier les conditions de la synthèse des méthylamines afin de déterminer les conditions optimales d'utilisation du catalyseur conforme à l'invention.

On opère dans les mêmes conditions qu'à l'exemple 4, en utilisant comme catalyseur la mordénite sous forme ammonium modifiée préparée à l'exemple 1, à 300 °C, mais en faisant varier le rapport atomique N/C et le débit d'alimentation du méthanol (nombre de kilogrammes de méthanol par heure par kilogramme de catalyseur).

Les résultats obtenus sont repris au tableau II dans lequel $C_{MeOH}$, $S_{DME}$, $S_{MMA}$, $S_{DMA}$ et $S_{TMA}$ ont la même signification qu'à l'exemple 4.

TABLEAU II

| Rapport atomique N/C | Débit de MeOH (kg/h/ kg de catalyseur) | $C_{MeOH}$ (% en moles) | $S_{DME}$ (% en moles) | $S_{MMA}$ (% en moles) | $S_{DMA}$ (% en moles) | $S_{TMA}$ (% en moles) |
|---|---|---|---|---|---|---|
| 1,3:1 | 0,2 | >99 | 0,8 | 16,2 | 77,0 | 6,0 |
| 1,3:1 | 0,3 | >99 | 0,6 | 17,7 | 81,6 | 0,1 |
| 1,6:1 | 0,2 | >99 | 0,7 | 21,1 | 76,2 | 2,0 |
| 1,6:1 | 0,3 | >99 | 0,5 | 21,3 | 77,7 | 0,4 |
| 2,0:1 | 0,2 | 99 | 0,4 | 25,5 | 72,5 | 1,6 |
| 2,0:1 | 0,3 | 97 | 0,4 | 27,2 | 72,1 | 0,3 |

On voit qu'à 300 °C et avec un débit d'alimentation du méthanol de 0,3 kg/h/kg de catalyseur, la production de triméthylamine est pratiquement nulle, et celle de diméthyléther est très faible, voisine de 0,5%, quel que soit le rapport atomique N/C des gaz d'alimentation. La sélectivité en diméthylamine peut atteindre 81,6 % en moles pour un rapport atomique N/C de 1,3. On observe aussi que la sélectivité en diméthylamine diminue lorsque le rapport atomique N/C augmente.

Exemple 6. Influence de la température de réaction.

On opère dans les mêmes conditions qu'à l'exemple 4 en utilisant comme catalyseur la mordénite sous forme ammonium modifiée préparée à l'exemple 1, excepté que l'on abaisse la température de réaction. Les résultats obtenus sont reportés au tableau III dans lequel $C_{MeOH}$, $S_{DME}$, $S_{MMA}$, $S_{DMA}$ et $S_{TMA}$ ont la même signification qu'à l'exemple 4.

TABLEAU III

| Température (°C) | Rapport atomique N/C | Débit de MeOH (kg/ h/kg de catalyseur) | $C_{MeOH}$ (% en moles) | $S_{DME}$ (% en moles) | $S_{MMA}$ (% en moles) | $S_{DMA}$ (% en moles) | $S_{TMA}$ (% en moles) |
|---|---|---|---|---|---|---|---|
| 280 | 1,6:1 | 0,1 | >98 | 0,5 | 20 | 76 | 3,5 |
| 260 | 1,6:1 | 0,1 | 64 | 2 | 46 | 52 | <0,1 |

Le tableau III montre qu'à plus basse température, c'est-à-dire à conversion incomplète du méthanol, les performances du catalyseur conforme à l'invention sont encore très bonnes; la formation de triméthylamine et de diméthyléther reste très faible.

Exemple 7. Stabilité du catalyseur.

On effectue la synthèse des méthylamines comme décrit à l'exemple 4 en utilisant comme catalyseur la mordénite sous forme ammonium modifiée préparée à l'exemple 1. Ce catalyseur est maintenu pendant 70 jours à 300 °C, à la pression atmosphérique, avec un rapport atomique N/C des gaz d'alimentation de 2:1 et un débit d'alimentation du méthanol de 0,2 kg/h/kg de catalyseur. Durant cette période, le catalyseur conserve son activité avec un taux de conversion du méthanol supérieur à 99 % en moles et une sélectivité en diméthylamine qui reste constante à 73 % en moles.

Exemple 8. Conversion de la monométhylamine.

Dans cet exemple, on étudie les performances du catalyseur conforme à l'invention dans la synthèse des méthylamines à partir d'un mélange gazeux d'ammoniac et de méthanol contenant de la monométhylamine.

On opère dans les mêmes conditions qu'à l'exemple 4 en utilisant comme catalyseur la mordénite sous forme ammonium modifiée préparée à l'exemple 1, à une température de réaction de 300 °C, mais on alimente au réacteur un mélange gazeux d'ammoniac, de méthanol et de monométhylamine en des quantités telles que le rapport atomique N/C soit de 2:1, avec un débit de méthanol de 0,1 kg/h/kg de catalyseur et un débit de monométhylamine de 0,2 kg/h/kg de catalyseur. Dans ces conditions, le taux de conversion du méthanol est supérieur à 99 % en moles et la sélectivité en diméthylamine est de 73 % en moles. On peut en conclure que la monométhylamine formée peut être recyclée aisément dans le mélange gazeux alimenté au réacteur et être transformée à son tour en diméthylamine.

Exemple 9.

Dans cet exemple, on étudie les performances du catalyseur conforme à l'invention dans la synthèse des méthylamines à partir d'un mélange gazeux d'ammoniac et de méthanol contenant de la triméthylamine.

On opère dans les mêmes conditions qu'à l'exemple 4, en utilisant comme catalyseur la mordénite sous forme ammonium modifiée préparée à l'exemple 1, à une température de réaction de 300 'C, mais on alimente au réacteur un mélange gazeux d'ammoniac, de méthanol et de triméthylamine en des quantités telles que le rapport atomique N/C soit de 2:1, avec un débit de méthanol de 0,1 kg/h/kg de catalyseur et un débit de triméthylamine de 0,067 kg/h/kg de catalyseur. Dans ces conditions, le taux de conversion du méthanol est de 100 % en moles, avec une sélectivité en diméthylamine de 70 % en moles. En outre. on retrouve à la sortie du réacteur la même quantité de triméthylamine qu'à l'entrée du réacteur.

**Revendications**

1. Procédé de préparation d'une mordénite sous forme ammonium modifiée, caractérisé en ce qu'il comprend

    (1) le séchage d'une mordénite sous forme ammonium à une température inférieure à 400°C, et
    (2) le traitement de la mordénite sous forme ammonium séchée ainsi obtenue par du tétrachlorosilane en phase gazeuse, à une température comprise entre 300 et 600°C.

2. Procédé selon la revendication 1, caractérisé en ce que l'étape de séchage (1) est exécutée dans l'intervalle de température de 230 à 350°C, pendant une durée de 180 à 540 minutes, de préférence de 360 à 480 minutes.

3. Procédé selon l'une quelconque des revendications 1 et 2, caractérisé en ce que l'étape de séchage (1) est effectuée sous une pression inférieure ou égale à 3 bars.

4. Procédé selon l'une quelconque des revendications 1 à 3,
   caractérisé en ce que l'étape de silylation (2) est exécutée sous la pression atmosphérique à une température de 450 à 550°C, pendant une durée de 60 à 180 minutes, de préférence de 120 à 160 minutes.

5. Procédé selon la revendication 1, caractérisé en ce que la mordénite sous forme ammonium modifiée possède un rapport atomique Si/Al de 10:1 à 30:1, de préférence de 15:1 à 25:1.

6. Catalyseur comprenant une mordénite sous forme ammonium modifiée obtenue par le procédé selon l'une quelconque des revendications 1 à 5.

7. Procédé de fabrication de méthylamines, caractérisé en ce que l'on fait passer en phase gazeuse à une température comprise entre 220°C et 500°C, un mélange de méthanol et d'ammoniac, sur un catalyseur comprenant une mordénite sous forme ammonium modifiée préparée par le procédé selon l'une quelconque des revendications 1 à 5.

8. Procédé selon la revendication 7, caractérisé en ce que le mélange de méthanol et d'ammoniac est utilisé en des quantités telles que le rapport atomique azote/carbone est de 0,5:1 à 5:1, de préférence de 0,8:1 à 2:1.

9. Procédé selon l'une quelconque des revendications 7 et 8, caractérisé en ce que le débit d'alimentation du méthanol est compris entre 0,1 et 2 kg/h/kg de catalyseur.

10. Procédé selon l'une quelconque des revendications 7 à 9, caractérisé en ce que la réaction est effectuée à une température comprise entre 220°C et 350°C, de préférence entre 280°C et 320°C.

11. Procédé selon l'une quelconque des revendications 7 à 10, caractérisé en ce que la réaction est effectuée sous une pression comprise entre la pression atmosphérique et 100 bars, de préférence entre 1 et 50 bars.

**12.** Procédé selon l'une quelconque des revendications 7 à 11, caractérisé en ce que le mélange de méthanol et d'ammoniac contient de la monométhylamine et/ou de la diméthylamine et/ou de la triméthylamine.

**Patentansprüche**

**1.** Verfahren zur Herstellung eines modifizierten Mordenits in Ammoniumform, dadurch gekennzeichnet, daß es

(1) die Trocknung eines Mordenits in Ammoniumform bei einer Temperatur unterhalb von 400°C und

(2) die Behandlung des auf diese Weise erhaltenen getrockneten Mordenits in Ammoniumform durch gasförmiges Tetrachlorsilan bei einer Temperatur zwischen 300 und 600°C

umfaßt.

**2.** Verfahren nach Anspruch 1, dadurch gekennzeichnet, daß der Schritt der Trocknung (1) in einem Temperaturbereich von 230 bis 350°C, während einer Dauer von 180 bis 540 Minuten, vorzugsweise von 360 bis 480 Minuten, ausgeführt wird.

**3.** Verfahren nach einem der Ansprüche 1 und 2, dadurch gekennzeichnet, daß der Schritt der Trocknung (1) unter einem Druck von unterhalb oder gleich 3 bar erfolgt.

**4.** Verfahren nach einem der Ansprüche 1 bis 3, dadurch gekennzeichnet, daß der Schritt der Silylierung (2) unter atmosphärischem Druck bei einer Temperatur von 450 bis 550°C, während einer Dauer von 60 bis 180 Minuten, vorzugsweise von 120 bis 160 Minuten, ausgeführt wird.

**5.** Verfahren nach Anspruch 1, dadurch gekennzeichnet, daß der modifizierte Mordenit in Ammoniumform ein Atomverhältnis Si/Al von 10:1 bis 30:1, vorzugsweise von 15:1 bis 25:1 aufweist.

**6.** Katalysator umfassend einen modifizierten Mordenit in Ammoniumform, erhalten durch ein Verfahren nach einem der Ansprüche 1-5.

**7.** Verfahren zur Herstellung von Methylaminen, dadurch gekennzeichnet, daß man in Gasphase bei einer Temperatur zwischen 220°C und 500°C ein Gemisch aus Methanol und Ammoniak über einen Katalysator leitet, der einen modifizierten Mordenit in Ammoniumform umfaßt, hergestellt durch ein Verfahren nach einem der Ansprüche 1-5.

**8.** Verfahren nach Anspruch 7, dadurch gekennzeichnet, daß das Gemisch aus Methanol und Ammoniak in solchen Mengen verwendet wird, daß das Atomverhältnis Stickstoff/Kohlenstoff 0,5:1 bis 5:1, vorzugsweise 0,8:1 bis 2:1 ist.

**9.** Verfahren nach einem der Ansprüche 7 und 8, dadurch gekennzeichnet, daß die Zufuhrmenge an Methanol zwischen 0,1 und 2 kg/h/kg Katalysator liegt.

**10.** Verfahren nach einem der Ansprüche 7 bis 9, dadurch gekennzeichnet, daß die Reaktion bei einer Temperatur zwischen 220°C und 350°C, vorzugsweise zwischen 280°C und 320°C erfolgt.

**11.** Verfahren nach einem der Ansprüche 7 bis 10, dadurch gekennzeichnet, daß die Reaktion unter einem Druck zwischen atmosphärischem Druck und 100 bar, vorzugsweise zwischen 1 und 50 bar erfolgt.

**12.** Verfahren nach einem der Ansprüche 7 bis 11, dadurch gekennzeichnet, daß das Gemisch aus Methanol und Ammoniak Monomethylamin und/oder Dimethylamin und/oder Trimethylamin enthält.

**Claims**

**1.** Process for preparing a mordenite in the modified ammonium form, characterized in that it comprises:

(1) drying a mordenite in the ammonium form at a temperature below 400°C, and

(2) treating the dried mordenite in the ammonium form so obtained with tetrachlorosilane in the gaseous phase, at a temperature of between 300 and 600°C.

2. Process according to claim 1, characterized in that the drying step (1) is carried out within the temperature interval 230 to 350°C, for a period of 180 to 540 minutes, preferably 360 to 480 minutes.

3. Process according to either of claims 1 or 2, characterized in that the drying step (1) is carried out under a pressure less than or equal to 3 bar.

4. Process according to any one of claims 1 to 3, characterized in that the silylation step (2) is carried out at atmospheric pressure at a temperature of 450 to 550°C, for a period of 60 to 180 minutes, preferably 120 to 160 minutes.

5. Process according to claim 1, characterized in that the mordenite in the ammonium form has an Si/Al atomic ratio of 10:1 to 30:1, preferably 15:1 to 25:1.

6. catalyst comprising a modified mordenite in the ammonium form obtained by the process according to any one of claims 1 to 5.

7. Process for manufacturing methylamines, characterized in that a mixture of methanol and ammonia is passed in the gaseous phase at a temperature of between 220°C and 500°C over a catalyst comprising a modified mordenite in the ammonium form prepared by the process according to any one of claims 1 to 5.

8. Process according to claim 7, characterized in that the mixture of methanol and ammonia is used in quantities such that the nitrogen/carbon atomic ratio is from 0.5:1 to 5:1, preferably from 0.8:1 to 2:1.

9. Process according to either of claims 7 or 8, characterized in that the methanol feed rate is between 0.1 and 2 kg/h/kg of catalyst.

10. Process according to any one of claims 7 to 9, characterized in that the reaction is carried out at a temperature of between 220°C and 350°C, preferably between 280°C and 320°C.

11. Process according to any one of claims 7 to 10, characterized in that the reaction is carried out under a pressure between atmospheric pressure and 100 bar, preferably between 1 and 50 bar.

12. Process according to any one of claims 7 to 11, characterized in that the mixture of methanol and ammonia contains monomethylamine and/or dimethylamine and/or trimethylamine.